# EUROPÄISCHE PATENTSCHRIFT

(11) **EP 2 094 285 B1**
(45) Veröffentlichungstag und Bekanntmachung des Hinweises auf die Patenterteilung: **08.07.2015**
(21) Anmeldenummer: 07857364.9
(22) Anmeldetag: 11.12.2007
(51) Int. Cl.: A61K 36/185, A61P 15/10

(54) **SPEZIALEXTRAKT UND SEINE VERWENDUNG ZUR HEMMUNG DES ABBAUS VON CYCLISCHEM GUANOSINMONOPHOSPHAT (CGMP)**
SPECIAL EXTRACT AND ITS USE TO INHIBIT THE DECOMPOSITION OF CYCLICAL GUANOSINMONOPHOSPHATE (CGMP)
EXTRAIT SPÉCIFIQUE ET SON UTILISATION POUR ATTÉNUER LA PERTE DE GUANOSINE MONOPHOSPHATE (CGMP) CYCLIQUE

(30) Priorität: 11.12.2006 EP 06125821
(43) Veröffentlichungstag der Anmeldung: 02.09.2009
(73) Patentinhaber: Finzelberg GmbH & Co. KG, 56626 Andernach (DE)
(72) Erfinder: FEISTEL, Björn, 56626 Andernach (DE); KREUTER, Matthias-Heinrich, 8880 Walenstadt (CH)
(74) Vertreter: Von Kreisler Selting Werner - Partnerschaft von Patentanwälten und Rechtsanwälten mbB
(86) Internationale Anmeldenummer: PCT/EP2007/063668
(87) Internationale Veröffentlichungsnummer: WO 2008/071684

(56) Entgegenhaltungen:
- WO-A-03/094943
- THARAKAN BINU ET AL: "Botanical therapies in sexual dysfunction" PHYTOTHERAPY RESEARCH, Bd. 19, Nr. 6, Juni 2005 (2005-06), Seiten 457-463, XP002438019 ISSN: 0951-418X in der Anmeldung erwähnt
- ARLETTI R ET AL: "Stimulating property of Turnera diffusa and Pfaffia paniculata extracts on the sexual behavior of male rats" PSYCHOPHARMACOLOGY, Bd. 143, Nr. 1, März 1999 (1999-03), Seiten 15-19, XP002438020 ISSN: 0033-3158 in der Anmeldung erwähnt
- ALARCON-AGUILAR F J ET AL: "INVESTIGATION ON THE HYPOGLYCAEMIC EFFECTS OF EXTRACTS OF FOUR MEXICAN MEDICINAL PLANTS IN NORMAL AND ALLOXAN-DIABETIC MICE" PHYTOTHERAPY RESEARCH, JOHN WILEY & SONS LTD. CHICHESTER, GB, Bd. 16, Nr. 4, Juni 2002 (2002-06), Seiten 383-386, XP008080184 ISSN: 0951-418X
- ANTONIO M A ET AL: "Oral anti-inflammatory and anti-ulcerogenic activities of a hydroalcoholic extract and partitioned fractions of Turnera ulmifolia (Turneraceae)" JOURNAL OF ETHNOPHARMACOLOGY, Bd. 61, Nr. 3, Juli 1998 (1998-07), Seiten 215-228, XP002439812 ISSN: 0378-8741

## Beschreibung

Die folgende Erfindung betrifft ein Verfahren zur Herstellung von Extrakten einer Pflanze aus der Familie der Turneraceae, die so erhaltenen Extrakte und ihre Verwendung.

Die WHO definiert das Menschenrecht in Bezug auf die Sexualität u.a. als Recht auf den höchsten erreichbaren Standard sexueller Gesundheit, sowie darauf, ein befriedigendes, sicheres und angenehmes Sexualleben führen zu können. (WHO Progress in Reproductive Health Research 2004; Nr. 67, pp. 3).

Nach einer globalen Studie ist für 84% der Männer Sexualität ein wichtiger Bestandteil ihres Lebens und wirkt sich ein befriedigendes Sexualleben positiv auf die empfundene Lebensqualität aus. Ein Großteil der Männer ab dem 40. Lebensjahr leiden an Erektionsstörungen = Erektile Dysfunktion (ED). Dies beschreibt die Unfähigkeit, eine Erektion zu erreichen oder aufrecht zu erhalten, die für ein befriedigendes Sexualleben ausreicht.

Ursachen für Erektionsstörungen können organischer und/oder psychischer Natur sein. Chronische Krankheiten, zunehmendes Alter, Medikamente und Genussmittel wie Alkohol und Nikotin dürfen ebenso als Risikofaktoren angenommen werden, wie Stress, Partnerschaftskonflikte, Depressionen oder Versagensangst. Durch den Verlust der Potenz wird das körperliche, seelische und soziale Selbstverständnis des Mannes, insbesondere des jungen Mannes, stark erschüttert. Patienten mit chronischer ED sind in ihrer Sexualität und Persönlichkeit derart verunsichert, das sie als krank anzusehen sind.

Um dieses Krankheitsbild zu verstehen, bedarf es der Grundlagen der Anatomie der Erektion: Der Penis enthält die dorsolateralen Schwellkörper Corpora cavernosa, sowie den medioventralen Schwellkörper Corpus spongiosum, welche schwammartige Gebilde aus kavernösen Räumen darstellen. Diese kavernösen Räume sind mit Gefäßepithelzellen ausgekleidet und werden über die Arteria pudenda interna mit Blut versorgt. Über die Vena dorsalis penis profunda fließt das Blut wieder ab.

Ebenso muss der Einfluss der Physiologie betrachtet werden. Erektion und Detumeszenz sind hämodynamische Vorgänge, welche über Relaxation und Kontraktion der Schwellkörpermuskulatur reguliert werden.

Im Ruhezustand liegt die sympathisch inervierte glatte Muskulatur der Schwellkörperarterien kontrahiert vor. Der Blutfluss durch die Schwellkörper ist daher minimal. Bei sexueller Stimulation steigt die parasympathische Aktivität, wodurch sich die Arterien weiten und der Blutdurchfluss der Schwellkörper ansteigt. Durch die Volumenzunahme werden die abführenden Venen zwischen Schwellkörpern und Tunica albuginea komprimiert und der Blutabfluss verringert. Die Folge dieses venooklusiven Mechanismus ist eine rigide Erektion.

Molekularphysiologisch kommt es zu folgendem Ablaufschema: Die sexuelle Stimulation führt über nonadrenerge / noncholinerge Nervenfasern zur L-Argininbasierten Produktion von Stickstoffmonoxid (NO). NO wirkt als "endothelium derived relaxing factor". Es diffundiert in die glatten Muskelzellen und aktiviert dort die Guanylatcyclase. Dies induziert die Bildung von cGMP aus GTP. cGMP führt zu einer Erschlaffung der glatten Schwellkörpermuskulatur. cGMP wird schließlich durch Phosphodiesterase Typ V (PDE-V) zu Guanosinmonophosphat (GMP) abgebaut und damit inaktiviert (Chew et al., Med. J. Aust. 172, 279-283, 2000).

Bei ED wird in vielen Fällen zu wenig NO in Relation zur benötigten Wirkung freigesetzt. Auch wird dies durch andere krankheitsbedingte Effekte wie Gefäßschädigungen oder erhöhter NO-Toleranz begünstigt. Der Weg über eine Steigerung der endothelial vermittelten NO-Produktion wird für viele "Heilmittel" beschrieben, oft in Zusammenhang mit der Gabe von L-Arginin als Kombinationspartner.

Der Zusammenhang von positiv getesteten NO-Donatoren (Bildung von cGMP) und der Auswirkung auf die Hemmung der PDE-V (Abbau von cGMP) ist nicht beschrieben.

Um nun eine stabile Erektion zu gewährleisten, bietet es sich ebenfalls an, den Abbaumechanismus des cGMP selektiv zu stören und somit eine längere Halbwertszeit des cGMP zu erreichen. Dieser Abbau wird durch die Phosphodiesterasen unterbunden. Um Nebenwirkungen weitgehend vermeiden zu können, hat es sich in der Vergangenheit als nützlich erwiesen, die Phosphodiesterase Typ V möglichst selektiv zu inhibieren, da diese fast ausschließlich in den Schwellkörpern vorhanden ist, während andere Subtypen wie PDE-I und PDE-II im Körper ubiquitär vorkommen. Dieser Wirkmechanismus ist für Sildenafil bekannt (Chew et al., Med. J. Aust. 172, 279 - 283, 2000).

Für diese synthetisch gewonnene Substanz ist neben dieser Wirkung der PDE-V-Hemmung auch eine ganze Reihe von Nebenwirkungen beschrieben. So sollte Sildenafil nicht kurz nach dem Essen oder nach Alkoholgenuss eingenommen werden, da sich die Wirkung dadurch deutlich vermindert, ebenfalls nicht bei Hypotonie, bekanntem Schlaganfallrisiko, Angina pectoris oder Herzinsuffiziens.

Für Sildenafil sind als Nebenwirkung unter anderem Blutdruckabfall, Kopfschmerzen, Dyspepsie und Muskelschmerzen beschrieben (Quelle: http://www.medhost.de/impotenz/sildenafil.html).

Von pflanzlichen Zubereitungen ist bekannt, dass sie ein sehr geringes Nebenwirkungsspektrum aufweisen. Auf der Suche nach Aphrodisiaka aus der Natur gelangt man schnell zu einer Vielzahl teils obskurer "Heilmittel", welche oft seit Jahrhunderten eingesetzt werden. Eine auf pflanzliche Präparate eingeschränkte Betrachtung der Thematik führt bei fast allen Naturvölkern immer noch zu einer Vielzahl unterschiedlicher verwendeter Pflanzen und Zubereitungen ünterschiedlichster Qualitäten bzw. Wirkstärken (Pflanzenpulver, Tee' s, Dekokte, Flüssig-Auszüge).

Aufgabe der vorliegenden Erfindung war es, eine bezüglich der ED wirksame Pflanze zu finden und deren wirksame Bestandteile in eine reproduzierbare Extraktform zu überführen. Somit ist eine therapeutisch gleichmäßige und effektive Dosis zu gewährleisten. Hierfür ist gegenüber dem ursprünglichen Pflanzenteil die Form eines hochkonzentrierten Pflanzenextraktes besonders geeignet.

Im Rahmen eines pharmakologischen in vitro Screenings wurden traditionell aphrodisierend angewandte Pflanzen hinsichtlich einer potentiellen PDE-V- Hemmung untersucht. Hierbei wurde eine diesbezüglich aktive Pflanzenfamilie entdeckt, die Safranmalvengewächse (Turneraceae).

Eine Pflanze dieser Gattung, die Turnera diffusa, deutsche Bezeichnung Damiana, ist originär heimisch am Golf von Mexiko und in der Karibik. Traditionell werden die getrockneten Laubblätter der Turnera diffusa und ihrer Varietäten zur Stärkung des Geschlechtstriebes in Form von Drogenpulver, Tee's oder auch Tonika konsumiert.

Ein solches Tonikum wurde durch Arletti et al. in Psycho Pharmacology (143, 1999, S. 15-19) beschrieben. Hiernach erweist sich ein traditioneller 1:1-Fluid im Tierversuch als wirksam bei lustlosen, impotenten Ratten. Ein Zusammenhang hinsichtlich der Art des Extraktes (Fluid-Extrakt: Einfluss des Ethanol-Anteils?) oder zur pharmakologischen Wirkungsweise wurde nicht herausgearbeitet. Tharakan et al. zitiert diese Arbeit in Phytotherapy Research (19, 2005, 457-463).

Pharmazeutisch verwendet werden in Deutschland ebenfalls traditionell alkpholisch-wässrige Einfachextrakte aus Damiana-Blättern, ohne jemals den wissenschaftlichen Beleg in Form einer Humanstudie erbracht zu haben (Handbuch der Phytotherapie, S. 106; Wiss. Verlagsgesellschaft Stuttgart 2003). Zu diesem Ergebnis kam bereits 1989 die Kommission E des früheren Bundesgesundheitsamtes, indem sie eine Negativ-Monographie vorlegte.

Es besteht daher immer noch ein Bedürfnis nach wirksamen pflanzlichen Extrakten, insbesondere zur Behandlung der erektilen Dysfunktion.

Gelöst wird die Aufgabe durch das erfindungsgemäße Verfahren zur Herstellung der Extrakte.

Völlig überraschenderweise wurde festgestellt, dass die Wirkungsstärke eines Damiana-Blattextraktes in Abhängigkeit gewählter Extraktions- und Verfahrensparameter für die erhaltenen Extrakte von nahezu inaktiv bis hochaktiv variierte. Ebenso unerwartet erwiesen sich sowohl Extraktionen mit rein hydrophilen Lösungsmitteln (Wasser) als auch Extraktionen mit hoch lipophilen Lösungsmitteln (reiner Ethanol) als ungeeignete Verfahrensweisen zur Erzielung einer hohen Aktivität in dem pharmakologischen Testsystem. Mittelpolare Auszugsmittel (z.B. Ethanol-Wasser Mischungen) zeigten vergleichsweise bessere Resultate.

Gegenstand der Erfindung ist daher zunächst ein Verfahren zur Herstellung eines Extraktes einer Pflanze aus der Familie der Turneraceae umfassend die Schritte
- Extraktion von Pflanzenteilen mit einem Extraktionsmittel, dass neben Wasser ein organisches Lösungsmittel ausgewählt aus Methanol, Ethanol, Propanol, Isopropanol, Aceton und Mischungen davon enthält
- Einengen des Extraktes zu einem Dickextrakt,
- Anreichern der lipophilen Substanzen zu einem Konzentrat.

Als Ausgangspflanzen eignen sich verschiedene Mitglieder der Familie der Turneraceae, insbesondere Turnera acaulis, Turnera acuta, Turnera alba, Turnera albicans, Turnera amapaensis, Turnera angustifolia, Turnera annectens, Turnera annularis, Turnera annularis var. conglomerata, Turnera aphrodisiaca, Turnera apifera, Turnera arcuata, Turnera arenaria, Turnera argentea, Turnera arillosa, Turnera armata, Turnera aromatica, Turnera aspera, Turnera asymmetrica, Turnera aturensis, Turnera urantiaca, Turnera aurea, Turnera aurelii, Turnera aurelioi, Turnera bahiensis, Turnera benthamiana, Turnera berneriana, Turnera bernieriana, Turnera etonicaefolia, Turnera blanchetiana var. aequalifolia, Turnera blanchetiana var. capituliflora, Turnera blanchetiana var. subspicata, Turnera blanchettana, Turnera brasiliensis, Turnera brasiliensis var. breviflora, Turnera brasiliensis var. brevifolia, Turnera breviflora, Turnera caatingana, Turnera caerulea var. surinamensis, Turnera callosa, Turnera calyptrocarpa, Turnera candida, Turnera capensis, Turnera capitata, Turnera capitata subsp. intermedia, Turnera caroliniana, Turnera carpinifolia, Turnera castilloi, Turnera chamaedrifolie, Turnera chamaedrys, Turnera chrysocephala, Turnera chrysodo-xa, Turnera cicatricosa, Turnera cipoensis, Turnera cistoides, Turnera clausenia-na, Turnera coerulea, Turnera collotricha, Turnera concinna, Turnera corchorifo-lia, Turnera corchoroides, Turnera coriacea, Turnera crulsii, Turnera cuneifolia, Turnera cuneiformis, Turnera curassavica, Turnera dasystyla, Turnera dasytricha, Turnera decipiens, Turnera desvauxii, Turnera dichotoma, Turnera dichotoma var. stenophylla, Turnera dichotoma var. stricta, Turnera diffusa, Turnera diffusa var. aphrodisiaca, Turnera diffusa var. diffusa, Turnera discolor, Turnera dolichostigma, Turnera duarteana, Turnera duarteana var. rotundifolia, Turnera eichleriana, Turnera elegans, Turnera elliptica, Turnera flammea, Turnera foliosa, Turnera frutescens, Turnera frutescens var. latifolia, Turnera gardneriana, Turnera genistoides, Turnera glabra, Turnera glaziovii, Turnera goyazensis, Turnera grandidentata, Turnera grandiflora, Turnera grandifolia, Turnera guia-nensis, Turnera harleyi, Turnera hassleriana var. lobulata, Turnera hatschbachii, Turnera hatschbachii var. miniata, Turnera hebepetala, Turnera helianthemoides, Turnera hermannioides, Turnera hexandra, Turnera hilaireana, Turnera hilaireana var. lanceolata, Turnera hilaireana var. minor, Turnera hilaireana var. oblongifo-lia, Turnera hilaireana var. ovatifolia, Turnera hindsiana, Turnera hindsiana subsp. brachyantha, Turnera hirsuta, Turnera hirsutissima, Turnera hirta, Turnera hispidissima, Turnera huberi, Turnera humboldtii, Turnera humifusa, Turnera ignota, Turnera incana, Turnera integrifolia, Turnera joelii, Turnera joel-lii, Turnera krapovickasii, Turnera lamiifolia, Turnera lanceolata, Turnera lep-tosperma, Turnera lineata, Turnera longiflora, Turnera longipes, Turnera lucida, Turnera luetzelburgii, Turnera luminosa, Turnera lutescens, Turnera macrophylla, Turnera madagascariensis, Turnera maracasana, Turnera marmorata, Turnera martii, Turnera melanorhiza, Turnera melanorhiza var. latifolia, Turnera melo-chia, Turnera melochioides, Turnera melochioides var. angustifolia, Turnera me-lochioides var. arenaria, Turnera melochioides var. genuina, Turnera melochioi-des var. oblongifolia, Turnera melochioides var. ramosissima, Turnera microphylla, Turnera mollis, Turnera muricata, Turnera nana, Turnera nervosa, Turnera oblongifolia, Turnera obtusifolia, Turnera oculata, Turnera oculta var. paucipilosa, Turnera odorata, Turnera opifera, Turnera orientalis, Turnera ovata, Turnera palmeri, Turnera panamensis, Turnera paniculata, Turnera paruana, Turnera parviflora, Turnera pernambucensis, Turnera peruviana, Turnera pilosu-la, Turnera pinifolia, Turnera pinnatifida, Turnera pinnatifida var. angustiloba, Turnera pinnatifida var. carnea, Turnera pinnatifida var. lycopifolia, Turnera poh-liana, Turnera prancei, Turnera princeps, Turnera pringlei, Turnera procumbens, Turnera pumila, Turnera pumilea, Turnera pumilea var. piauhyensis, Turnera pumileoides, Turnera purpurascens, Turnera racemosa, Turnera ramosissima, Turnera refracta, Turnera revoluta, Turnera riedeliana, Turnera rosea, Turnera rubrobracteata, Turnera rugosa, Turnera rupestris, Turnera rupestris var. frutes-cens, Turnera salicifolia, Turnera schomburgkiana, Turnera schwackeana, Turnera sedoides, Turnera selloi, Turnera sericea, Turnera serrata, Turnera ser-rata var. angustifolia, Turnera serrata var. brevifolia, Turnera serrata var. latifo-lia, Turnera serrata var. schwackei, Turnera setifera, Turnera setosa, Turnera setosa var. entreriana, Turnera setosa var. integrifolia, Turnera sidaefolia, Turnera sidoides, Turnera sidoides subsp. carnea, Turnera sidoides subsp. holo-sericea, Turnera sidoides subsp. integrifolia, Turnera sidoides subsp. pinnatifida, Turnera sidoides var. angustiloba, Turnera sidoides var. grisebachiana, Turnera sidoides var. herteriana, Turnera sidoides var. hispida, Turnera sidoides var. holosericea, Turnera sidoides var. incisa, Turnera sidoides var. lycopifolia, Turnera simulans, Turnera stachydifolia, Turnera stachydifolia var. flexuosa, Turnera stenophylla, Turnera steyermarkii, Turnera stipularis, Turnera subglabra, Turnera subnuda, Turnera subulata, Turnera surinamensis, Turnera tapajoensis, Turnera tenuicaulis, Turnera thomasii, Turnera tomentosa, Turnera tortuosa, Turnera triglandulosa, Turnera trigona, Turnera trioniflora, Turnera uleana, Turnera ulmifolia, Turnera ulmifolia var. acuta, Turnera ulmifolia var. alba, Turnera ulmifolia var. coerulea, Turnera ulmifolia var. cuneiformis, Turnera ulmi-folia var. elegans, Turnera ulmifolia var. elliptica, Turnera ulmifolia var. grandi-dentata, Turnera ulmifolia var. grandiflora, Turnera ulmifolia var. intermedia, Turnera ulmifolia var. orientalis, Turnera ulmifolia var. velutina, Turnera urbanii, Turnera valleana, Turnera velutina, Turnera venosa, Turnera villosa, Turnera violacea, Turnera virgata, Turnera viscosa, Turnera waltherioides, Turnera wed-delliana, Turnera weddelliana var. brachyphylla, Turnera weddelliana var. norma-lis, Turnera whitei, Turnera xanthotricha, Turnera zanthotricha oder Turnera zeasperma. Eine bevorzugte Gattung sind die Pflanzen aus der Gattung Turnera diffusa WILLD, insbesondere Turnera diffusa var. aphrodisiaca Urb.

Als Pflanzenteile sind insbesondere Blätter oder Kraut geeignet, die für eine Pflanzenextraktion typisch in geschnittener oder gemahlener Form eingesetzt werden.

Als Extraktionsmittel werden wässrige Lösungen von wassermischbaren organischen Lösungsmitteln, ausgewählt aus Ethanol, Methanol, Propanol, Isopropanol, Aceton und Mischungen davon, eingesetzt.

Bevorzugt enthält das Extraktionsmittel das Lösungsmittel oder die Lösungsmittelmischung in einer Menge von 20 bis 90, bevorzugt 30 bis 80 und noch mehr bevorzugt 50 bis 70 Gew.-%. Der Rest des Extraktionsmittels ist Wasser.

Bevorzugt wird die primäre Extraktion erschöpfend durchgeführt, dabei kann die Extraktion bei erhöhter Temperatur beispielsweise 30 bis 50°C, bevorzugt 40 bis 50°C durchgeführt werden.

Auch mehrstufige Extraktionen können eingesetzt werden.

Pro Gewichtsteil zu extrahierender Pflanzenteile haben sich Mengen an 1 bis 20 Gewichtsteile, bevorzugt 6 bis 12 Gewichtsteile Extraktionsmittel als besonders geeignet erwiesen.

Nach der Extraktion wird der erhaltene Extrakt zu einem Dickextrakt eingeengt. Dieses kann durch dem Fachmann bekannte Methoden geschehen, beispielweise einer Beidampfung im Vakuum am Rotationsverdampfer, an einem Dünnfilmverdampfer oder an einem Plattenverdampfer.

Ein entscheidender Schritt zur Erhöhung der Wirksamkeit des Extrakts ist ein anschließender Schritt zur Anreicherung lipophiler Substanzen, verbunden mit der Abreicherung hydrophiler Substanzen.

Als besonders geeignet hierfür hat sich ein Verfahren erwiesen, bei dem zu dem Dickextrakt Wasser zugegeben wird, der Dickextrakt dispergiert wird und der wässrige Überstand verworfen wird. Der Rückstand, das sogenannte Extraktivstoff-Präzipitat, wird durch eine Reextraktion mit einem Gemisch aus organischem Lösungsmittel und Wasser von unlöslichen Bestandteilen abgetrennt. Die so gewonnenen Extraktivstoffe werden beigedampft, mit Trocknungshilfstoffen versetzt und getrocknet.

Als besonders geeignet hat es sich erwiesen, für die Dispergierung des Dickextraktes die Menge an Wasser so auszuwählen, dass auf 100 Gewichtsteile Wasser etwa 2 bis 12 Gewichtsteile des Dickextraktes eingesetzt werden.

Ein anderes Verfahren zur Anreicherung der lipophilen Substanzen in dem Konzentrat und damit zur Abreicherung der hydrophilen Substanzen ist eine Flüssig/Flüssig-Extraktion, mit der eine Verteilung der Inhaltsstoffe zwischen Wasser und einem nicht mit Wasser löslichen, lipophilen Lösungsmittel erreicht wird. Die wässrige Phase wird verworfen und der lipophilere Flüssig-Extrakt weiter verwendet.

Eine Anreicherung lipophiler Extrakte ist auch durch chromatographische Methoden zu erreichen, bei der lipophile Komponenten beispielsweise an RP-Materialien gebunden werden und die nicht gebundenen Substanzen entfernt werden, bevor die gebundenen Substanzen eluiert werden.

Das oben beschriebene Verfahren des Dispergierens des Dickextraktes in Wasser ist besonders bevorzugt.

Aus der bevorzugten Ausführungsform des Dispergierens geht ein ExtraktivstoffPräzipitat hervor, das in einer wässrigen Lösung mit einem organischem Lösungsmittel von 30 bis 90 Gew.-%, ausgewählt aus Methanol, Ethanol, Propanol, Isopropanol, Aceton und Mischungen davon, reextrahiert wird. Die hiernach erhältlichen Extraktivstoffe werden von den ungelösten Bestandteilen separiert und zu einem Spissumextrakt beigedampft.

Zu einem erfindungsgemäßen Extrakt, der an lipophilen Komponenten vermindert ist, werden bevorzugt Trockenhilfsstoffe gegeben und das so erhaltene Gemisch getrocknet. Trotz des relativ hohen co-extrahierten Chlorophyllgehaltes im Extrakt werden hierdurch Trockenextrakte erhalten, die gut zu verarbeiten sind. Als Trocknungshilfsstoffe haben sich insbesondere solche aus Polysacchariden wie beispielsweise Gummi arabicum, Xanthan Gum, etc. sowie Trockenhilfsstoffe auf Basis kristalliner Cellulose und Siliziumdioxid als geeignet erwiesen.

In einer besonders bevorzugten Ausführungsform werden pro Gewichtsteil des erhaltenen Rohextraktes 0,1 bis 0,6 Gewichtsteile an Gummi arabicum, 0,05 bis 0,5 Gewichtsteile an mikrokristalliner Cellulose und 0,01 bis 0,1 Gewichtsteile an Siliziumdioxid als Trockenhilfsstoffe eingesetzt.

Gegenstand der Erfindung ist auch der durch das erfindungsgemäße Verfahren erhältliche Extrakt, insbesondere in Form eines Trockenextraktes. Dieser zeigt eine deutliche Hemmung der humanen Phosphodiesterase V.

Bevorzugt liegt der IC-50 Wert gemäß dem in den Beispielen beschrieben Verfahren bei 4 bis 10 µg/mL.

Gegenstand der Erfindung ist weiterhin die Verwendung des erfindungsgemäßen Extraktes zur Herstellung einer Zubereitung, insbesondere eines Arzneimittels zur Behandlung von Zuständen, bei denen eine Erhöhung des zellulären cGMP-Spiegels durch einen verzögerten Abbau von cGMP gewünscht wird.

Der Extrakt eignet sich damit insbesondere zur Herstellung einer Zubereitung, insbesondere eines Arzneimittels zur Behandlung erektiler Dysfunktionen, insbesondere soweit die erektile Dysfunktion durch einen organselektiven Mangel an cGMP verursacht ist.
Figur 1 zeigt die PDE-V Hemmung der erfindungsgemäßen Extrakte im Vergleich zu Sildenafil gemäß Beispiel 5.
Figur 1a) zeigt die PDE-V-Hemmung der Sildenafil-Kontrolle.
Figur 1b) zeigt die PDE-V-Hemmung des erfindungsgemäßen Damiana-Spissumextraktes ohne Trocknungshilfstoffe [Extraktmuster 5-1].
Figur 1c) zeigt die PDE-V-Hemmung des erfindungsgemäßen Damiana - Trockenextraktes [Extraktmuster 5-2]

Für beide Extrakte wurden jeweils die gleichen Mengen an nativem Trockenextraktequivalent für den PDE-V Test verwendet, sie sind daher direkt vergleichbar.
Figur 2 zeigt schematisch die Herstellung gemäß Beispiel 3.
Figur 3 und Figur 4 zeigen dünnschichtchromatographische Analysen von Extrakten.

Die Erfindung wird durch die nachfolgenden Beispiele näher erläutert.

### Beispiel 1: PDE-V Hemmung in Abhängigkeit vom Extraktionsmittel

Zunächst wurde die Hemmung der Phosphodiesterase V in Abhängigkeit der Lösemittelkonzentration getestet. Dazu wurden Dickextrakte aus Folia Damiana mit den unten angegebenen Lösungsmitteln im Droge-Lösungsmittelverhältnis 1:12 bei 40°C hergestellt, eingeengt und die Extrakte auf ihre Hemmung der PDE-V getestet. Das Testsystem war wie folgt charakterisiert - PDE-V Assay (Durchführung nach Schilling, R.J. et al.: Anal. Biochem. 216, 154-158 (1994) und Mullershausen F. et al.: J. Cell Biology 160, 719-727 (2003):

Nach Vorinkubation der Assaymischung (PDE-V- Proteingehalt 32 mg/ml) mit den Extraktproben bei 24°C für 15 min, wurde die Reaktion durch Zugabe des Substrates [³H]-cGMP Tracer (0.003 µCi/µl) gestartet. Anschließend wurde die Assaymischung bei 30°C für 20 min inkubiert. Nullkontrollen t₍₀₎ wurden ohne Zugabe von Enzym durchgeführt.

IC₅₀-Werte wurden unter Zuhilfenahme eines Tritium Scintillation Proximity Assays (PDE-[³H]-cGMP SPA Amersham Biosciences TRKQ 7100) gemäß den Vorschriften des Herstellers Amersham ermittelt. Die Messwerte für die Proben wurden aus mind. zwei Versuchen je Konzentration ermittelt.

Dabei ergaben sich die in Tabelle angegebenen Werte

**Tab. 1 - Hemmung der PDE-V in Abhängigkeit der Lösemittelauswahl (Testkonzentration der Extrakte 0,3 / 3,0 / 30,0 µg/mL; alle auf native Trockenextraktequivalente bezogen)**

| Auszugsmittel | Wasser | EtOH 30% m/m | EtOH 70% m/m | EtOH 99% m/m |
|---|---|---|---|---|
| IC ₅₀ µg/mL | > 30 * | 15 - 20 | 10 - 15 | > 30 * |

| | | | | |
|---|---|---|---|---|
| * Keine Inhibierung in diesem Konzentrationsbereich messbar. | | | | |

### Beispiel 2: Variation der Turnera Species

Basierend auf diesen Erkenntnissen wurde versucht, eine näherer Differenzierung der in der Literatur aufgeführten Turnera Species zu den im Handel geführten Qualitäten von Blättern der Turneraceen gegenüberzustellen. Verschiedene Species und Varietäten von Turnera diffusa wurden hinsichtlich Ihrer PDE-V Aktivität geprüft. Hierzu wurden Extrakte aus deren Blättern gemäß den Extraktionsangaben von Bsp.1 mit Ethanol 60 % m/m extrahiert.

**Tab. 2 - Hemmung der PDE-V in Abhängigkeit der Turnera-Species; (Testkonzentration der Extrakte 0,3 / 3,0 / 30,0 µg/mL ; auf native Trockenextraktäquivalente bezogen)**

| Turnera Species | T. aphrodisiaca | T. diffussa var. diffusa | T. diffussa var. aphodisiaca | T. ulmifolia |
|---|---|---|---|---|
| IC ₅₀ µg/mL | 10 - 15 | 10 - 20 | 10 - 15 | 10-20 |

### Beispiel 3: Aufreinigung von lipophilen Bestandteilen

Im Rahmen von weiteren Arbeiten wurde dann überraschenderweise gefunden, dass durch eine mehrstufige Prozessführung, beginnend mit einer Primärextraktion mit einem Wasser-Ethanolgemisch von 60 - 80% m/m, einer anschließenden Beidampfung zum Dickextrakt, einer folgenden Präzipitation in wässrigem Milieu und Separation des Überstandes, anschließender Reextraktion des Präzipitates mit einem Wasser-Ethanolgemisch von 40-60 %m/m, ein Spezialextrakt resultierte, der eine gegenüber dem Primärextrakt mehrfach erhöhte Aktivität und gegenüber rein lipophilen Extrakten eine rund 10-fach erhöhte Aktivität im pharmakologischen Testsystem der Phosphodiesterase V Hemmung aufwies.

20 kg Folia Damiana werden erschöpfend mit 60% m/m Ethanol bei 40°C im Perkolator extrahiert. Die Eluate werden von der Droge getrennt, filtriert und am Plattenverdampfer bei max. 50°C zu einem lösemittelfreien Spissum eingeengt. Es resultierten 5800 g mit einem Trockensubstanzanteil von 60,3% (= Extraktstufe A).

331,7 g des obigen Spissumextraktes (= 200 g natives Trockenextraktäquivalent) werden in demineralisiertem Wasser unter Rühren portionsweise dispergiert. Dabei wird die Menge an Wasser so gewählt, das der Trockenrückstand der entstehenden Mischung bei 10% m/m liegt. Diese Mischung wird für 1 Stunde weiter gerührt und anschließend über 12 Stunden bei Raumtemperatur stehen gelassen. Das ausgefallene Extraktivstoff-Präzipitat wurde vom Überstand abgetrennt.

Die Lösung des Überstandes wurde filtriert und beigedampft. Es resultierten 216 g Spissum mit einem Arbutingehalt von 3,8% (bezogen auf nativen Trockenextrakt) und mit einem Trockensubstanzanteil von 60% (= Extraktstufe B). Das so erhaltene, unbehandelte Extraktivstoff-Präzipitat wurde homogenisiert (Extraktstufe C).

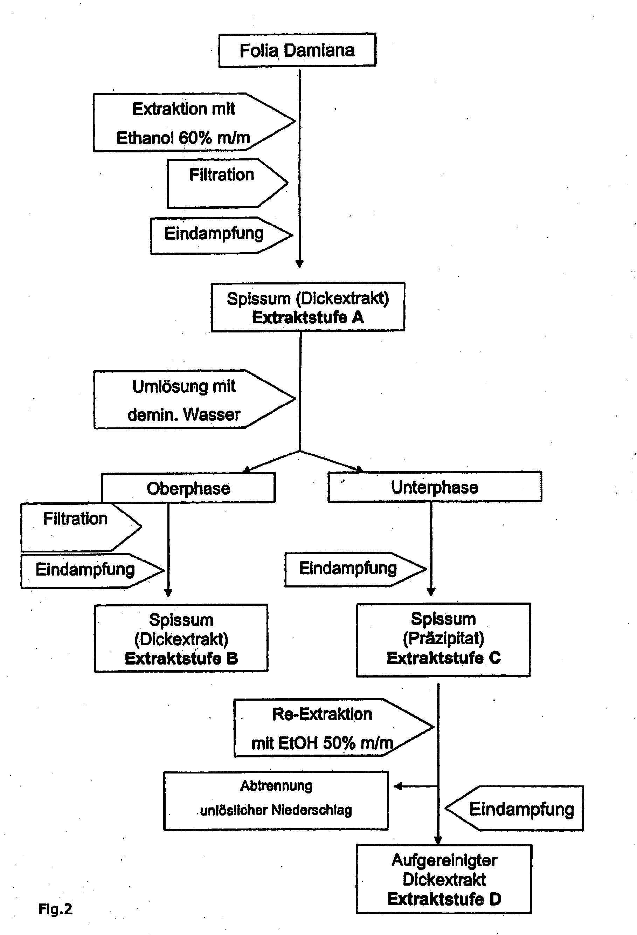

Das Extraktivstoff-Präzipitat wird anschließend mit Ethanol 50% m/m im Verhältnis 1:5 reextrahiert. Dabei fällt neben den löslichen Extraktivstoffen ein unlöslicher Niederschlag an, der verworfen wird. Die gewonnenen Extraktivstoffe werden klarfiltriert und zu einem ethanolisch-wässrigen Flüssig-Extrakt beigedampft. Es resultierten 284 g Flüssig-Extrakt mit einem Arbutingehalt von 2,2% (bezogen auf nativen Trockenextrakt) und mit einem Trockensubstanzanteil von 23% (= Extraktstufe D). Dies ist der erfindungsgemäße Extrakt. Der Ablauf ist in Figur 2 schematisch dargestellt.

**Tab. 3 - Hemmung der PDE-V in Abhängigkeit der Extraktionsstufen (Testkonzentration der Extrakte 0,3 / 3,0 / 30,0 µg/mL ; auf native Trockenextraktäquivalente bezogen)**

| Extraktstufen | A | B | C | D |
|---|---|---|---|---|
| IC ₅₀ µg/mL | 15 | 15 | 10 | 4 |

### Beispiel 4: Trockenhilfsstoffe

Der so erhaltene Flüssigextrakt lässt sich allerdings weder mittels Routineverfahren zu einem homogenen Spissumextrakt aufkonzentrieren, noch zu einem Pulver trocknen, da bei diesem Herstellweg eine Vielzahl von lipohilen Substanzen wie z.B. Chlorophyll mitextrahiert wird, was bekanntlich zu Ausfällungen und Phasentrennungen in Spissumextrakten führt. Nur die Überführung in einen Trockenextrakt und dessen Mahlung kann zu einem homogenen Produkt führen. Überraschend zeigte sich jedoch der Einfluss verschiedener Trocknungshilfsstoffe.

Während mit üblichen Trocknungshilfsstoffen der Trocknungsprozess ausreichend erfolgreich gelingt, konnte nur eine Verschlechterung oder annähernde gleichbleibende Wirkung auf die PDE-V-Hemmung festgestellt werden.

Durch Zusatz von einem Gemisch einer wässrigen Lösung von Gummi arabicum und dem Hilfsstoff Prosolv™ (silizilierte mikrokristalline Cellulose), während des Beidampfungsprozesses gelang es, das Auftreten von Phasentrennungen und Ausfällungen zu verhindern und nach Konzentrierung zum Spissumextrakt, das erhaltene Extrakt-Hilfsstoff-Gemisch zu trocknen und ein freifließendes Pulver zu gewinnen.

750 kg Folia Damiana werden erschöpfend mit 60% m/m Ethanol bei 45°C im Perkolator extrahiert. Die Eluate werden von der Droge getrennt, filtriert und am Plattenverdampfer bei max. 50°C zu einem lösemittelfreien Spissum eingeengt. Es resultierten 250 kg mit einem Trockensubstanzanteil von 74,9%

1335 g des obigen Spissumextraktes (= 1000 g natives Trockenextraktäquivalent) werden in demineralisiertem Wasser unter Rühren portionsweise dispergiert. Dabei wird die Menge an Wasser so gewählt, das der Trockenrückstand der entstehenden Mischung bei 5% m/m liegt. Diese Mischung wird für 1 Stunde weiter gerührt und anschließend über 12 Stunden bei Raumtemperatur stehen gelassen. Das ausgefallene Extraktivstoff-Präzipitat wurde vom Überstand abgetrennt und anschließend mit Ethanol 50% m/m im Verhältnis 1:4 reextrahiert. Unlöslichen Bestandteile wurden abgetrennt und verworfen. Die so gewonnenen Extraktivstoffe werden klarfiltriert und zu einem ethanolisch-wäßrigen Flüssig-Extrakt beigedampft. Der resultierende Flüssig-Extrakt wurde mit den Trocknungshilfsstoffen in gelöster bzw. suspendierter Form versetzt, homogenisert und lösemittelfrei beigedampft und unter Vakuum bei 50°C getrocknet.

**Tab. 4 - Hemmung der PDE-V in Abhängigkeit der eingesetzten Trocknungshilfsstoffe (70% Nativanteil / 30% Hilfsstoffe); (Testkonzentration der Extrakte 0,3 / 3,0 / 30,0 µg/mL ; auf native Trockenextraktäquivalente bezogen)**

| Zusammensetzung der Damiana-Extraktzubereitung | Flüssigextrakt ohne Hilfsstoffe | 70% Nativ / 25 % GA / 5% PVP | 70% Nativ / 25 % GA / 5% Prosolv™ | 70% Nativ / 25 % MD / 5% Prosolv™ | 70% Nativ / 25 % PVPP / 5% Prosolv™ |
|---|---|---|---|---|---|
| IC ₅₀ µg/mL | 6 | 7 | 4 | 6 | 6 |

| | | | | | |
|---|---|---|---|---|---|
| GA - Gummi arabicum PVP - Lösliches Polyvidon PVPP - Unlösliches Polyvidon Prosolv™ - silizilierte, mikrokristalline Cellulose MD - Maltodextrin | | | | | |

### Beispiel 5: Prüfung der PDE-V Hemmung

Die Prüfung im pharmakologischen Testsystem zeigt, dass durch die erfindungsgemäßen Verfahrensschritte und Prözess-Parameter ein Spezialextrakt herstellbar ist, der sich gegenüber herkömmlichen Extrakten durch eine mehrfach erhöhte Aktivität gegenüber der Phosphodiesterase V auszeichnet. Die Erfindung ermöglicht zusätzlich die Umwandlung des erhaltenen Flüssigextraktes in einen lagerstabilisierten Trockenextrakt.

750 kg Folia Damiana werden erschöpfend mit 60% m/m Ethanol bei 45°C im Perkolator extrahiert. Die Eluate werden von der Droge getrennt, filtriert und am Plattenverdampfer bei max. 50°C zu einem lösemittelfreien Spissum eingeengt. Es resultierten 250 kg mit einem Trockensubstanzanteil von 74,9%

2003 g des obigen Spissumextraktes (= 1500 g natives Trockenextraktäquivalent) werden in demineralisiertem Wasser unter Rühren portionsweise dispergiert. Dabei wird die Menge an Wasser so gewählt, das der Trockenrückstand der entstehenden Mischung bei 5% m/m liegt. Diese Mischung wird für 1 Stunde weiter gerührt und anschließend über 12 Stunden bei 4°C stehen gelassen. Das ausgefallene Extraktivstoff-Präzipitat wurde vom Überstand abgetrennt und anschließend mit Ethanol 50% m/m im Verhältnis 1:1 reextrahiert. Unlösliche Bestandteile wurden abgetrennt und verworfen. Die so gewonnenen Extraktivstoffe werden klarfiltriert und zu einem ethanolisch-wäßrigen Flüssig-Extrakt beigedampft. Es resultierten 8900 g (41% Trockensubstanzanteil) lipophiler Flüssigextrakt (= Extraktmuster 5-1).

Der resultierende Flüssig-Extrakt wurde anteilig mit den Trocknungshilfsstoffen Gummi arabicum (25%) und Prosolv™ (5%) in gelöster bzw. suspendierter Form versetzt, homogenisert und lösemittelfrei beigedampft und unter Vakuum bei 50°C getrocknet (Extraktmuster 5-2).

Die Prüfung im PDE-V-Assay ergab folgende IC 50 - Konzentrationen (alle Angaben bezogen auf native Trockenextraktequivalente):
Extraktmuster 5-1 = 8 µg/mL
Extraktmuster 5-2 = 5 µg/mL

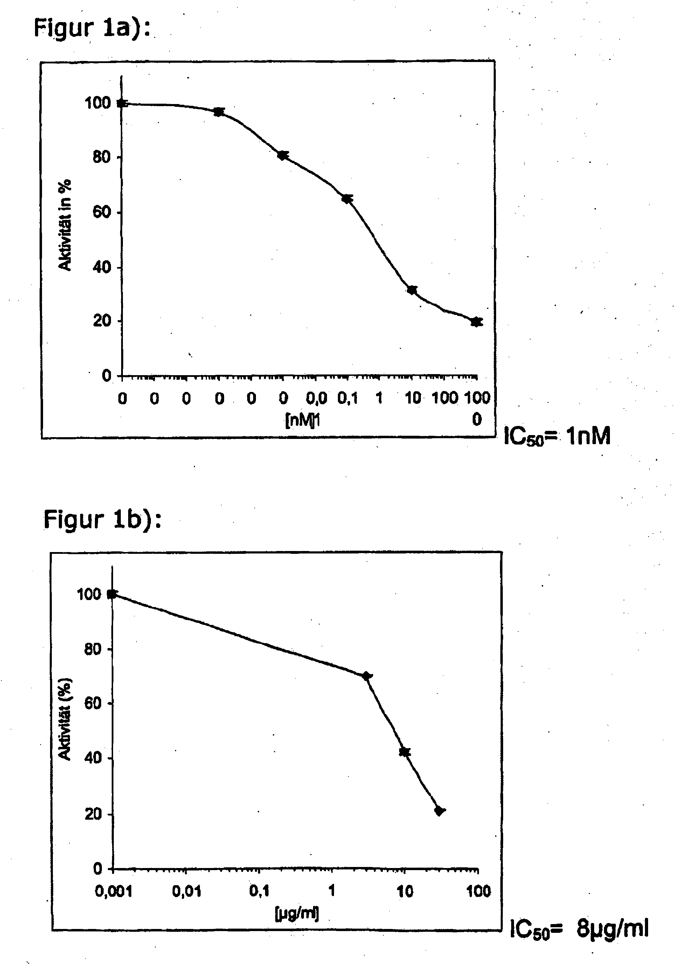

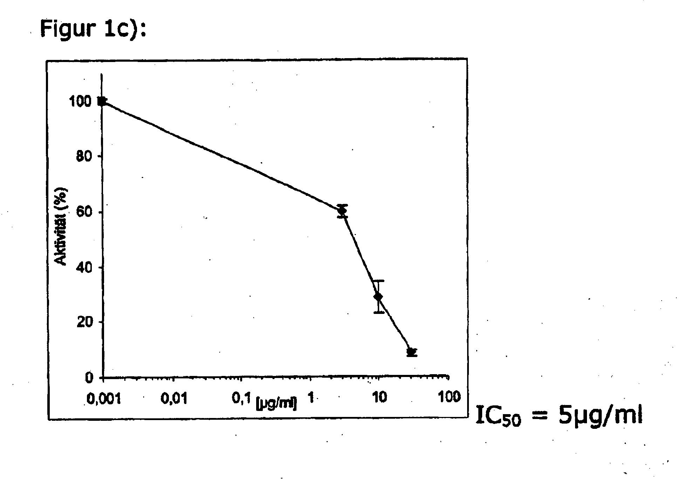

Die graphische Auswertung dieser PDE-V-Hemmung im Vergleich zu Sildenafil ist in der Figur 1 dargestellt.

### Beispiel 6: Vergleichsextrakt

Zunächst wurde ein Vergleichsextrakt gemäß Arletti et al. hergestellt. Hierzu wurden Blätter mit Ethanol 30% (v/v) bei Raumtemperatur über Nacht mazeriert. Das Extraktionsmittel wurde abgetrennt und der Rückstand ausgepresst. Die ausgepresste Flüssigkeit wurde mit dem Extrakt vereint. Es erfolgte eine Einengung zu einem Trockenextrakt und Rücklösen zu einem Fluidextrakt (1:1).

### Beispiel 7: Dünnschichtchromatographische Analyse

Der Extrakt von Arletti gemäß Beispiel 6 und der erfindungsgemäße Extrakt gemäß Beispiel 3 wurden dünnschichtchromatographisch untersucht.

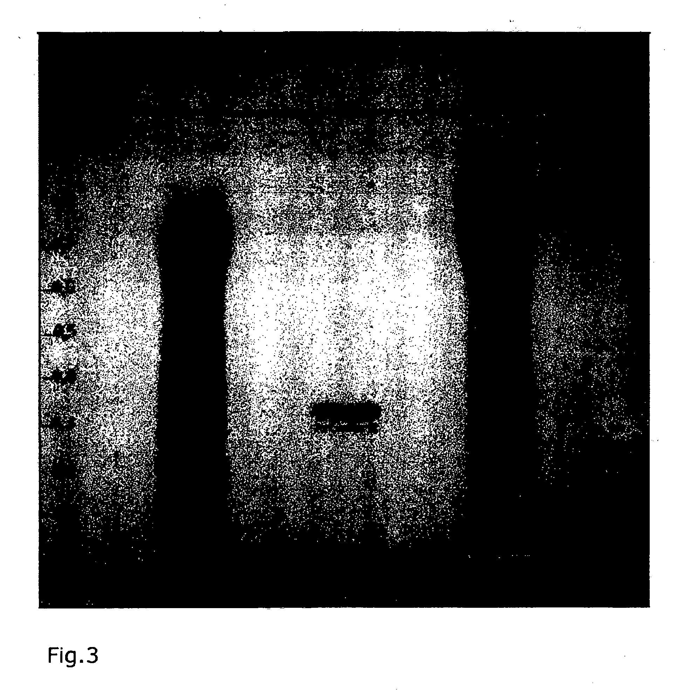

Figur 3 zeigt eine chromatographische Untersuchung auf Hydrochinon-Derivate (Kieselgel 60 F₂₅₄, wasserfreie Ameisensäure:Wasser: Ethylacetat (6:6:88, v/v/v)) nach Bedampfen mit Ammoniaklösung. Bahn 1 zeigt den Extrakt von Arletti, Bahn 3 den erfindungsgemäßen Extrakt.

Es zeigt sich, dass der Arletti-Extrakt einen höheren Anteil hydrophiler Substanze hat, während dieser Anteil bei dem erfindungsgemäßen Extrakt gering ist.

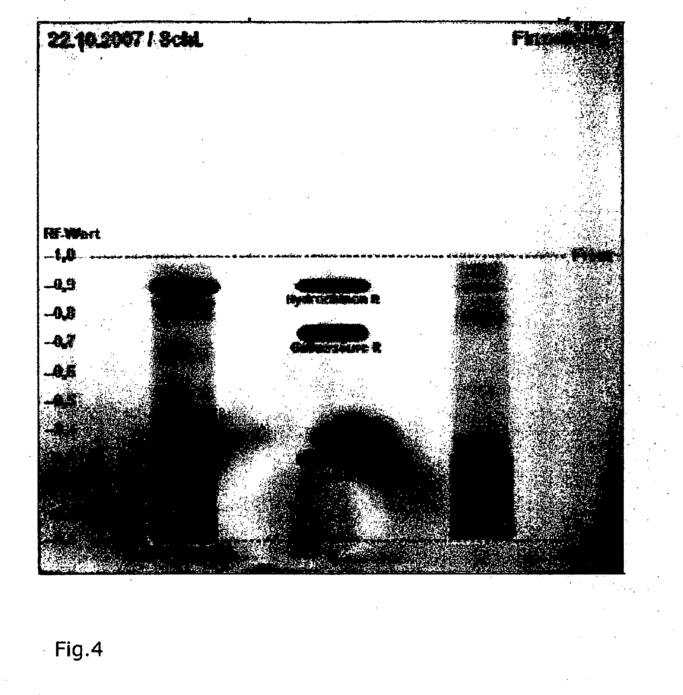

Figur 4 zeigt eine dünnschichtchromatographische Untersuchung auf polare Inhaltsstoffe (Kieselgel 60 F₂₅₄, Dichlormethan:Essigsäure:Methanol:Wasser (50:25:15:15 v/v/v/v) Sprühreagenz: Anisaldehyd. Bahn 1 ist der Extrakt nach Arletti, Bahn 3 der erfindungsgemäße Extrakt. Es zeigt sich der stärkere lipophile Anteil im erfindungsgemäßen Extrakt.

### Beispiel 8: Vergleich der PDE-5-Hemmung

Der Extrakt gemäß Beispiel 6 (Arletti) und der erfindungsgemäße Extrakt wurden in Konzentrationen von 1, 3, 10, 30 und 60 µg/ml in einem PDE-5-Assay getestet. Hierzu wurde PDE-5 (Proteingehalt 13,5 µg/ml) mit den Extrakten bei 24°C für 10 min vorinkubiert. Anschließend wurde die Reaktion durch Zugabe von ³H-GMP (0,003 µCi/µl) gestartet. Die Assay-Mischung wurde für 20 min bei 30°C inkubiert. Nullkontrollen wurden ohne Zugabe von PDE-V durchgeführt. Die IC₅₀-Werte wurden unter Zuhilfenahme eines Tricium Scintillation Proximity Assays (PDE- [³H]-cGMP; Amersham Biosciences TRKQ 7100) gemäß den Vorschriften des Herstellers ermittelt.

Die Messwerte für die Proben wurden aus zwei Versuchen ermittelt und sind als Mittelwerte mit jeweiliger Abweichung vom Mittelwert angegeben. Die dosisabhängige Reduktion der Enzymaktivität unter Einwirkung der jeweiligen Probe oder Referenzsubstanz ist ausgedrückt in einer prozentualen Dosis-Wirkungskurve. Die IC₅₀-Werte (Probenkonzentration, die eine 50% Inhibition der maximalen Enzymaktivität bewirkt) wurden mit dem Programm GraphPad-Prism (Version 4, GraphPad Software Inc., San Diego, CA, USA) bestimmt.

Vergleichsextrakt gemäß Beispiel 6:IC₅₀ = 18,5 µg/ml (95% Vertrauensintervall 17,63 bis 19,32 µg/ml).

Erfindungsgemäßer Extrakt: IC₅₀ 2,5 µg/ml (95% Vertrauensintervall 2,13 bis 2,86 µg/ml).

Um eine 50%ige Inhibierung des Enzyms PDE-V zu erreichen, wird vom erfindungsgemäßen Extrakt eine deutlich geringere Substanzmenge benötigt. Der erfindungsgemäße Extrakt ist um den Faktor 7,4 stärker.

## Patentansprüche

1. Verfahren zur Herstellung eines Extraktes aus einer Pflanze aus der Familie der Turneraceae umfassend die Schritte
- Extraktion von Pflanzenteilen mit einem Extraktionsmittel, dass neben Wasser ein organisches Lösungsmittel ausgewählt aus Methanol, Ethanol, Propanol, Isopropanol, Aceton und Mischungen davon, enthält
- Einengen des Extraktes zu einem Dickextrakt,
- Anreichern lipophiler Substanzen zu einem Konzentrat.

2. Verfahren nach Anspruch 1, **dadurch gekennzeichnet, dass** die Anreicherung lipophiler Substanzen zu einem Konzentrat
- durch Dispergieren des Dickextraktes in Wasser und Abtrennen und Verwerfen des wässrigen Überstandes, um ein Extraktivstoff-Präzipitat zu erhalten, Reextraktion des Extraktivstoff-Präzipitates und Separation ungelöster Substanzen von gelösten Extraktivstoffen oder
- durch eine Flüssig/Flüssig-Extraktion oder
- durch chromatographische Methoden
erfolgt.

3. Verfahren nach Anspruch 2, **dadurch gekennzeichnet, dass** das Dispergieren ein Extraktivstoff-Präzipitat hervorbringt, das in einer wässrigen Lösung mit einem Lösungsmittel von 30 bis 90 Gew.-% ausgewählt aus Methanol, Ethanol, Propanol, Isopropanol, Aceton und Mischungen davon, reextrahiert und mindestens die in diesem Lösemittelgemisch löslichen Extraktivstoffe von ungelösten Bestandteilen separiert und gewonnen werden.

4. Verfahren nach Anspruch 2, **dadurch gekennzeichnet, dass** das Dispergieren ein Extraktivstoff-Präzipitat hervorbringt, das in einer wässrigen Lösung mit Ethanol 40 bis 60 Gew.-% reextrahiert wird und die in diesem Lösemittelgemisch löslichen Extraktivstoffe von ungelösten Bestandteilen separiert und gewonnen werden.

5. Verfahren nach Anspruch 1 bis 4, **dadurch gekennzeichnet, dass** die Pflanzenteile Blätter oder Kraut sind, wobei die Blätter oder das Kraut in geschnittener oder gemahlener Form extrahiert werden.

6. Verfahren nach mindestens einem der Ansprüche 1 bis 5, **dadurch gekennzeichnet, dass** das Extraktionsmittel das organische Lösungsmittel in einer Menge von 20 bis 90, bevorzugt 50 bis 70 Gew.-% enthält.

7. Verfahren nach mindestens einem der Ansprüche 1 bis 6, **dadurch gekennzeichnet, dass** pro Gewichtsteil zu extrahierender Pflanzenteile 1 bis 20 Gewichtsteile, bevorzugt 6 bis 12 Gewichtsteile Extraktionsmittel eingesetzt werden.

8. Verfahren nach mindestens einem der Ansprüche 1 bis 7, **dadurch gekennzeichnet, dass** das erhaltene Konzentrat mit Trocknungshilfsstoffen versetzt wird und dieses Gemisch getrocknet wird, wobei vorzugsweise die Trocknungshilfsstoffe ausgewählt werden aus Polysacchariden, wie Gummi arabicum, Xanthan Gum oder kristallinen Cellulosen und/oder Siliziumdioxiden.

9. Verfahren nach Anspruch 8, **dadurch gekennzeichnet, dass** pro Gewichtsteil an gewonnenen Extraktivstoffen von 0,1 bis 0,6 Gewichtsteile an Gummi arabicum, und 0,05 bis 0,5 Gewichtsteile an mikrokristalliner Cellulose und 0,01 bis 0,1 Gewichtsteile an Siliziumdioxid eingesetzt werden.

10. Extrakt, erhältlich gemäss dem Verfahren nach einem der Ansprüche 1 bis 9.

11. Extrakt nach Anspruch 10 in Form eines Trockenextraktes.

12. Extrakt nach Anspruch 10 oder 11, **dadurch gekennzeichnet, dass** dieser Extrakt eine Hemmung der humanen Phosphodiesterase V (PDE-5) bewirkt.

13. Verwendung eines Extraktes nach mindestens einem der Ansprüche 10 bis 12 zur Herstellung einer Zubereitung zur Behandlung erektiler Dysfunktionen.

14. Verwendung eines Extraktes nach mindestens einem der Ansprüche 10 bis 12 zur Herstellung einer Zubereitung zur Behandlung von Sehschwäche, Diabetes oder zur organselektiven Regeneration der glatten Muskulatur.

## Claims

1. A process for the preparation of an extract from a plant of the Turneraceae family, comprising the steps of:
- extracting plant parts with an extractant comprising, in addition to water, an organic solvent selected from methanol, ethanol, propanol, isopropanol, acetone and mixtures thereof;
- concentrating the extracts to form a viscous extract;
- enriching the lipophilic substances to form a concentrate.

2. The process according to claim 1, **characterized in that** the enrichment of lipophilic substances into a concentrate is effected
- by dispersing the viscous extract in water and separating and discarding the aqueous supernatant to obtain an extractive precipitate, re-extracting the extractive precipitate and separating undissolved substances from dissolved extractives; or
- by a liquid/liquid extraction; or
- by chromatographic methods.

3. The process according to claim 2, **characterized in that** said dispersing yields an extractive precipitate, which is re-extracted in an aqueous solution with a solvent of from 30 to 90% by weight selected from methanol, ethanol, propanol, isopropanol, acetone and mixtures thereof, and at least the extractives soluble in this mixture of solvents are separated from undissolved components and recovered.

4. The process according to claim 2, **characterized in that** said dispersing yields an extractive precipitate, which is re-extracted in an aqueous solution with from 40 to 60% by weight ethanol, and the extractives soluble in this mixture of solvents are separated from undissolved components and recovered.

5. The process according to claim 1 to 4, **characterized in that** said plant parts are leaves or herb, the leaves or herb being extracted in a cut or ground form.

6. The process according to at least one of claims 1 to 5, **characterized in that** said extractant contains said organic solvent in an amount of from 20 to 90%, preferably from 50 to 70%, by weight.

7. The process according to at least one of claims 1 to 6, **characterized in that** from 1 to 20 parts by weight, preferably from 6 to 12 parts by weight, of extractant is employed per part by weight of plant parts to be extracted.

8. The process according to at least one of claims 1 to 7, **characterized in that** the obtained concentrate is admixed with drying auxiliaries, and the mixture obtained is dried, wherein preferably said drying auxiliaries are selected from polysaccharides, such as gum arabic, xanthan gum or crystalline celluloses and/or silicon dioxides.

9. The process according to claim 8, **characterized in that** from 0.1 to 0.6 part by weight of gum arabic, from 0.05 to 0.5 part by weight of microcrystalline cellulose and from 0.01 to 0.1 part by weight of silicon dioxide are employed per part by weight of extractives obtained.

10. An extract obtainable by the process according to any of claims 1 to 9.

11. The extract according to claim 10 in the form of a dry extract.

12. The extract according to claim 10 or 11, **characterized in that** said extract causes inhibition of human phosphodiesterase V (PDE-5).

13. Use of an extract according to at least one of claims 10 to 12 for preparing a formulation for the treatment of erectile dysfunctions.

14. Use of an extract according to at least one of claims 10 to 12 for preparing a formulation for the treatment of impaired vision, diabetes, or for the organ-selective regeneration of smooth muscles.

## Revendications

1. Procédé de fabrication d'un extrait d'une plante de la famille des Turneraceae, comprenant les étapes suivantes :
- extraction de parties de plante avec un agent d'extraction qui, outre de l'eau, contient un solvant organique sélectionné parmi le méthanol, l'éthanol, le propanol, l'isopropanol, l'acétone et des mélanges de ceux-ci,
- réduction de l'extrait en un extrait épais,
- enrichissement de substances lipophiles en un concentré.

2. Procédé selon la revendication 1, **caractérisé en ce que** l'enrichissement de substances lipophiles en un concentré s'effectue
- par la dispersion de l'extrait épais dans de l'eau et la séparation et le rejet du résidu aqueux afin d'obtenir un précipité de matières extractives, réextraction du précipité de matières extractives et séparation de substances non dissoutes de matières extractives dissoutes, ou
- par une extraction liquide-liquide ou
- par des méthodes chromatographiques.

3. Procédé selon la revendication 2, **caractérisé en ce que** la dispersion donne un précipité de matières extractives qui est ré-extrait dans une solution aqueuse avec un solvant de 30 à 90 % en poids sélectionné parmi le méthanol, l'éthanol, le propanol, l'isopropanol, l'acétone et des mélanges de ceux-ci, et **en ce que** au moins les matières extractives solubles dans ce mélange de solvants sont séparées de constituants non dissous et collectées.

4. Procédé selon la revendication 2, **caractérisé en ce que** la dispersion donne un précipité de matières extractives qui est ré-extrait dans une solution aqueuse avec de l'éthanol à 40 à 60 % en poids, et **en ce que** les matières extractives solubles dans ce mélange de solvants sont séparées de constituants non dissous et collectées.

5. Procédé selon les revendications 1 à 4, **caractérisé en ce que** les parties de plantes sont des feuilles ou de l'herbe, les feuilles ou l'herbe étant extraites sous forme découpée ou moulue.

6. Procédé selon au moins une des revendications 1 à 5, **caractérisé en ce que** l'agent d'extraction contient le solvant organique à raison d'une quantité de 20 à 90, de préférence de 50 à 70 % en poids.

7. Procédé selon au moins une des revendications 1 à 6, **caractérisé en ce que** pour chaque partie en poids de parties de plante à extraire, 1 à 20 parties en poids, de préférence 6 à 12 parties en poids d'agent d'extraction sont mises en oeuvre.

8. Procédé selon au moins une des revendications 1 à 7, **caractérisé en ce que** le concentré obtenu est mélangé avec des agents de séchage, et **en ce que** ce mélange est séché, les agents de séchage étant de préférence sélectionnées parmi des polysaccharides tels que la gomme arabique, la gomme xanthane ou des celluloses cristallines et/ou des dioxydes de silicium.

9. Procédé selon la revendication 8, **caractérisé en ce que** pour chaque partie en poids de matières extractives obtenues, de 0,1 à 0,6 parties en poids de gomme arabique, et de 0,05 à 0,5 parties en poids de cellulose microcristalline et de 0,01 à 0,1 parties en poids de dioxyde de silicium sont mises en oeuvre.

10. Extrait pouvant être obtenu conformément au procédé selon une des revendications 1 à 9.

11. Extrait selon la revendication 10 sous la forme d'un extrait sec.

12. Procédé selon la revendication 10 ou 11, **caractérisé en ce que** cet extrait provoque une inhibition de la phosphodiestérase V humaine (PDE-5).

13. Utilisation d'un extrait selon au moins une des revendications 10 à 12 pour la fabrication d'une préparation destinée au traitement de dysfonctionnements érectiles.

14. Utilisation d'un extrait selon au moins une des revendications 10 à 12 pour la fabrication d'une préparation destinée au traitement de la baisse de l'acuité visuelle, du diabète ou destinée à la régénération organosélective des muscles lisses.
